(19) Europäisches Patentamt
European Patent Office
Office européen des brevets

(11) Numéro de publication: **0 477 618 A2**

(12) **DEMANDE DE BREVET EUROPEEN**

(21) Numéro de dépôt: **91114990.4**

(22) Date de dépôt: **05.09.91**

(51) Int. Cl.[5]: **C07C 1/20**, C07C 1/36, C07C 11/21, C07D 305/12, C07C 59/42

(30) Priorité: **25.09.90 CH 3087/90**

(43) Date de publication de la demande: **01.04.92 Bulletin 92/14**

(84) Etats contractants désignés: **CH DE FR GB LI NL**

(71) Demandeur: **FIRMENICH SA**
**1, route des Jeunes**
**CH-1211 Genève 8(CH)**

(72) Inventeur: **Fehr, Charles**
**6, chemin Ravoux**
**CH-1290 Versoix(CH)**
Inventeur: **Galindo, José**
**7, chemin Croix-du-Levant**
**CH-1220 Les Avanchets(CH)**

(74) Mandataire: **Salvadori, Giuseppe, Dr.**
**c/o Firmenich S.A. Case Postale 239**
**CH-1211 Genève 8(CH)**

(54) **Procédé pour la préparation d'une oléfine polyinsaturée.**

(57) L'undéca-1,3,5-triène, ingrédient parfumant prisé, est préparé par un procédé en 2, respectivement 3 étapes à partir d'acide sorbique.

Le procédé présente l'avantage de permettre l'obtention d'undéca-1,3,5-triène sous sa forme isomérique préférentielle d'undéca1,3E,5Z-triène.

EP 0 477 618 A2

La présente invention a trait au domaine de l'industrie de la parfumerie. En particulier, elle a pour objet un procédé pour la préparation d'un composé oléfinique polyinsaturé, l'undéca-1,3,5-triène, ingrédient parfumant fort apprécié.

Depuis sa découverte [voir : Chrétien-Bessière et al., Bull. Soc. Chim. Fr 1967, 97], nombreuses ont été les voies de synthèse proposées pour sa préparation [voir par exemple : brevet FR 74 19580 ; brevet AS 68 01077 ; demande de brevet EP 203 615 ; F. Naef et al., Helv. Chim. Acta 1975, 58, 1016 ; V. Ratovelomanana et al., Bull. Soc. Chim. Fr. 1987, 174 ; Recherches 1967, 16, 5 ; W. Boland et al., Helv. Chim. Acta 1987, 70, 1025 ; E. Block et al., J. Am. Chem. Soc 1986, 108, 4568]. Certaines parmi les méthodes proposées ont trouvé une application industrielle et l'undéca-1,3,5-triène est à présent commercialisé sous des dénominations diverses. Caractérisé par la présence dans sa molécule de trois doubles liaisons éthyléniques, l'undéca-1,3,5-triène peut se présenter sous différentes formes isomériques dont les proportions respectives dans le produit final déterminent sa qualité olfactive. Or, nous avons pu constater qu'aucun des procédés décrits dans l'art antérieur pouvait tout à la fois satisfaire aux exigences d'économie, de sécurité ou de respect de l'environnement et offrir un produit de qualité irréprochable, supérieure à celle du produit présentement offert sur le marché.

Le procédé de l'invention présente l'avantage de permettre l'obtention d'undéca-1,3,5-triène sous forme d'un mélange isomérique dont le contenu en l'isomère undéca-1,3E,5Z-triène est prépondérant. Or c'est précisément cet isomère qui présente au mieux les propriétés odorantes les plus caractéristiques ; sa présence anoblit donc les caractères du mélange.

Le procédé de l'invention est caractérisé en ce qu'on

a. additionne de l'hexanal au sel dilithié de i'acide sorbique de formule

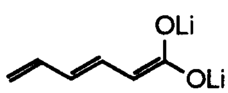

(I)

pour fournir un acide $\beta$-hydroxylique de formule

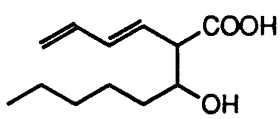

(II)

et

b. soumet ledit acide $\beta$-hydroxylique à une réaction d'élimination par traitement avec un réactif constitué par un N,N-diméthylformamidedialkylacétal ou par l'adduit constitué par la triphénylphosphine et l'ester diéthylique de l'acide azodicarboxylique,

ou

c. convertit ledit acide $\beta$-hydroxylique en la lactone de formule

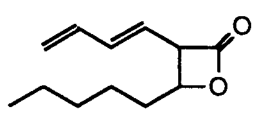

(III)

par traitement avec le chlorure d'acétyle, l'anhydride acétique ou un chlorure d'arènesulfonyle en présence d'une base ;

et

d. soumet la lactone ainsi obtenue à un traitement thermique à une température comprise entre 250 et 300 ° C.

Le procédé défini ci-dessus est illustré à l'aide du schéma réactionnel suivant :

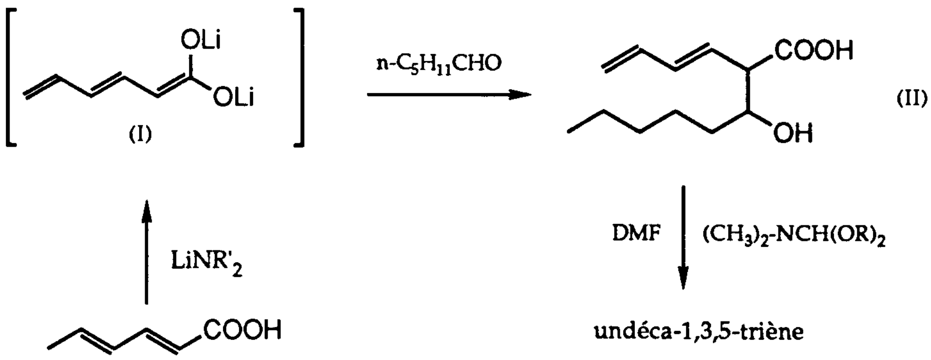


R et R' = alkyle

ou :

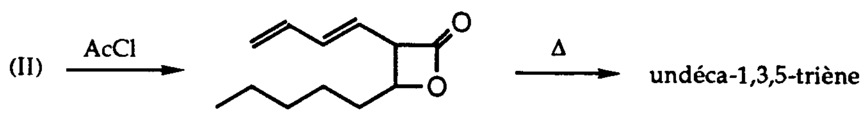


(III)

Ac = acétyle

Tel que mentionné plus haut, l'avantage principal du procédé de l'invention réside dans le fait qu'il permet l'obtention d'undéca-1,3,5-triènesous forme d'un mélange isomérique préférentiel dans lequel la teneur en undéca-1,3E,5Z-triène est plus élevée que celle de l'isomère 1,3E,5E.

Un tel effet peut s'expliquer par le fait que l'addition de l'hexanal sur le di-anion formé par le sel dilithié de l'acide sorbique (I) a lieu en position $\alpha$ pour donner un mélange *anti/syn* d'hydroxy-acide (II), dont le rapport respectif des deux isomères varie en fonction de la température à laquelle on effectue la réaction d'addition.

Ainsi, des températures basses, au voisinage par exemple de 0 ° C, favorisent la formation du diastéréomère *anti*, tandis que des températures plus élevées, par exemple de l'ordre de 50 ° C, tendent à favoriser l'obtention de l'isomère *syn* :

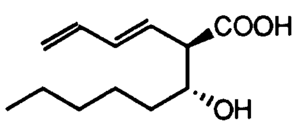

(II) -*anti*

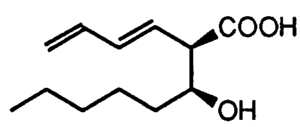

(II)-*syn*

De façon inattendue, nous n'avons pas observé la formation de produits qui seraient dus à une addition en position terminale $\epsilon$.

L'étape suivante du procédé de l'invention consiste en une élimination carboxy-hydroxylative, laquelle peut être effectuée à l'aide des réactifs connus dans l'art pour promouvoir des fragmentations similaires [voir à cet effet : Hara et al., Tetrahedron Lett. 1975, 1545 ; Mulzer et Brüntrup, Angew. Chem. 1977, 89, 265 ; Mulzer et Lammar, Angew. Chem. 1983 95, 629 ; Mulzer et al., J. Chem. Soc. Chem. Commun. 1979, 52 ; Rüttimann et al., Helv. Chim. Acta 1975, 58, 1450]. Parmi de tels réactifs figurent les dialkylacétals du N,N-diméthylformamide, en particulier le N,N-diméthylformamide-diméthylacétal et le N,N-diméthylformamidedinéopentylacétal, ainsi que l'adduit constitué par l'ester diéthylique de l'acide azodicarboxylique avec la triphénylphosphine :

$EtO_2C-N = N-CO_2Et/Ph_3P$.

La réaction de fragmentation est effectuée de préférence dans le diméthylformamide.

Selon une variante du procédé de l'invention, l'acide $\beta$-hydroxylique (II), sous la forme d'un mélange *anti/syn*, est transformé en lactone (III) au moyen d'une réaction avec des réactifs tel un chlorure

d'arènesulfonyle, comme le chlorure de benzènesulfonyle, un chlorure acide, tel le chlorure d'acétyle, ou l'anhydride acétique.

La réaction de lactonisation s'effectue en présence d'une base. Or, nous avons observé que par l'emploi, à titre de base, d'une trialkylamine telle la triéthylamine, il y avait formation préférentielle de la lactone (III) sous forme d'un mélange isomérique dans lequel la proportion en l'isomère cis était prépondérante (env. 70:30). Cette proportion des deux isomères

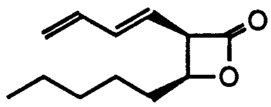

(III)-cis

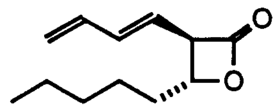

(III)-trans

était par ailleurs indépendante de la proportion respective ***anti/syn*** de l'acide $\beta$-hydroxylé de départ. Ce résultat est d'autant plus surprenant que la littérature fait état du caractère hautement ***syn***-sélectif de la lactonisation des acides $\beta$-hydroxylés au moyen du système benzènesulfonylchlorure/pyridine [voir références indiquées plus haut].

C'est ainsi que la lactone (III) a pu être préparée sous forme d'un mélange isomérique avec un contenu d'environ 70:30 en partant indifféremment par exemple d'un mélange 2:1 ou 1:4 d'***anti/syn*** $\beta$-hydroxy acide.

La dernière étape du procédé selon la variante décrite consiste en un traitement thermique de la lactone obtenue, lequel traitement peut être conduit selon les méthodes habituelles, par exemple par pyrolyse à une température comprise entre 250° et 300° C, de préférence au voisinage de 280° C, des vapeurs de lactone (III). Un tube à pyrolyse en quartz convient parfaitement à cette opération. Selon un mode opératoire préférentiel, la lactone est dissoute au préalable dans un solvant approprié, par exemple l'acétate d'éthyle, puis sous un courant de gaz: inerte, tel l'azote ou l'argon, les vapeurs sont pyrolysées à 280° C dans un tube en quartz d'environ 4m. Le pyrolysat est condensé par la suite pour fournir l'undéca-1,3,5-triène désiré, le cas échéant après purification et stabilisation.

Le sel dilithié de l'acide sorbique (I), utilisé comme produit de départ dans le procédé de l'invention, peut être obtenu par traitement de l'acide sorbique avec le diisopropylamidure de lithium ou avec tout réactif analogue.

L'acide $\beta$-hydroxylique (II) ainsi que la lactone (III) sont des composés chimiques de structure nouvelle. La présente invention a également pour objet lesdits composés, à savoir
l'acide (1RS,2SR)-2-(1-hydroxyhexyl)-3,5-hexadiénoïque,
l'acide (1RS,2RS)-2-(1-hydroxyhexyl)-3,5-hexadiénoïque,
la (3RS,4SR)-(E)-3-(1,3-butadiényl)-4-pentyl-2-oxétanone et
la (3RS,4RS)-(E)-3-(1,3-butadiényl)-4-pentyl-2-oxétanone.

La présente invention sera illustrée à l'aide de l'exemple suivant dans lequel les températures sont indiquées en degrés centigrades et les abréviations ont le sens usuel dans l'art.

Exemple

Méthode A

a. Un mélange constitué par le sel dilithié de l'acide sorbique a été préparé de la façon suivante.

Dans un ballon de 1,5 l équipé d'agitation mécanique et maintenu sous atmosphère d'argon, on a additionné rapidement à 38° du styrène (3,2g ; 0,031 mole) à une suspension de granulés de lithium (4,24g ; 0,606 mole) dans 61,2 g (0,606 mole) de diisopropylamine et 400 ml de tétrahydrofuranne (THF). Après 10 min, la réaction étant amorcée, on a additionné lentement pendant 45 min une solution de styrène (28,4 g ; 0,273 mole) dans 200 ml de THF. A la fin de l'introduction, le mélange réactionnel a été chauffé pendant une heure supplémentaire à 38°. La solution obtenue a été ensuite refroidie à -10° et 29,7 g (0,265 mole) d'acide sorbique dans 200 ml de THF ont été ajoutés tout en maintenant la température à une valeur inférieure à -2°.

Après 15 min, on a ajouté au mélange réactionnel pendant 20 min 35,8 g d'hexanal (0,358 mole) tandis que la température a été maintenue entre -10° et -2°. Après 15 min, le mélange réactionnel froid a été versé dans de l'eau glacée (250 ml) puis dilué avec 150 ml de pentane, ce qui a permis une bonne

séparation des phases. La phase organique séparée a été lavée avec 200 ml d'eau puis les phases aqueuses combinées ont été extraites avec de l'éther et acidifiées avec de l'acide sulfurique à 10% (150 ml). On a extrait ensuite deux fois à l'éther et lavé les phases organiques combinées avec de l'eau et une solution aqueuse saturée de NaCl. On a séché sur $Na_2SO_4$, filtré et concentré. On a ainsi obtenu 56,1 g d'un mélange des acides (1RS,2SR)-2-(1-hydroxyhexyl)-3,5-hexadiénoïque et (1RS,2RS)-2-(1-hydroxyhexyl)-3,5-hexadiénoïque (rend. : 100%). Une distillation au four à boules d'un échantillon de 5g dudit mélange à 150-190° (température du four/1,33 Pa) a fourni 3,63 g (72%) d'un mélange des acides dont le contenu en isomère ***anti*** par rapport à l'isomère ***syn*** était de 2:1.

L'isomère (1RS,2SR) présentait les caractères analytiques suivants :

| | |
|---|---|
| IR ($CHCl_3$) : | 3700-2400, 2930, 1700, 1400, 1275 $cm^{-1}$ |
| $^1$H-RMN(360MHz) : | 0,88(3H,t,J=7) ; 1,17-1,62(8H,m) ; 3,12(1H,t,J=9); 3,86(1H,m) ; 5,12-(1H,d,J=10) ; 5,22(1H,d,J=16) ; 5,65(1H,dxd,J=9 et 14,5) ; 6,25(1H,m) ; 6,32(1H,m) ; env. 6,0-6,5(2H,large) δ ppm |
| $^{13}$C-RMN(360MHz) : | 14,0(q) ; 22,6(t) ; 25,1(t) ; 31,7(t) ; 34,6(t) ; 56,0(d) ; 72,7(d) ; 118,1(t) ; 127,5(d) ; 135,5(d) ; 136,1(d) ; 177,6(s) δ ppm |
| SM : | 194(3), 123(11), 112(77), 97(78), 94(36), 81(35), 67(91), 55(65), 44(92), 41-(100), 39(60). |

L'isomère (1RS,2RS) présentait les caractères analytiques suivants :

| | |
|---|---|
| $^1$H-RMN(360MHz) : | 3,12(dxd,1H,J=13 et 3,5) ; 4,00(1H,m); 5,80(1H,dxd,J=9 et 14,5) δ ppm |
| $^{13}$C-RMN(360MHz) : | 25,3(t) ; 34,1(t) ; 71,9(d) ; 117,9(d) ; 126,1(d) ; 136,2(d) ; 136,4(d) ; 178,4(s) δ ppm |

b. 7,01 g (33,1 mmole) des acides obtenus comme indiqué ci-dessus en solution dans 50 ml de diméthylformamide (DMF) ont été ajoutés goutte à goutte à 50° à une solution agitée de 4,34 g (4,85 ml; 36,4 mmole) de diméthylformamide-diméthylacétal dans 20 ml de DMF. Une fois l'addition terminée (45 min), l'évolution de $CO_2$ a cessé et le mélange réactionnel a encore été agité pendant 15 min puis versé dans un mélange glace/eau. On a extrait trois fois à l'éther de pétrole et les extraits organiques combinés ont été lavés à l'eau, avec une solution d'HCl aqueux à 5%, avec du bicarbonate de sodium et enfin avec une solution aqueuse saturée de NaCl. L'évaporation de la phase organique a permis l'obtention de 4,82 g d'undéca-1,3,5-triène brut, produit qui a été stabilisé par l'adjonction de BHA. Par distillation fractionnée de ce produit, on a obtenu le produit désiré sous forme d'un mélange 58:42 des isomères trans,cis-1,3,5- et trans,trans-1,3,5-undecatriène, Eb. 80-82°/1,06x$10^3$ Pa. Un mélange desdits acides β-hydroxyliques a été préparé en suivant la méthode indiquée ci-dessus mais, après addition de l'hexanal, le mélange réactionnel a été chauffé pendant 3h à 50°. Le rendement des acides obtenus est de 100%. Toutefois, dans ce cas, le contenu respectif des isomères ***anti/syn*** est de 1:4.

Méthode B

c. Une solution des acides β-hydroxyliques obtenus comme indiqué plus haut (40,1 g; pureté : env. 80%) dans 750 ml de toluène a été traitée à 0° avec 57,3 g (79 ml ; 567 mmole) de triéthylamine. On a additionné ensuite en 45 min une solution de 11,9 g (10,75 ml ; 151 mmole) de chlorure d'acétyle dans 25 ml de toluène. On a maintenu la température à +2° et on a agité encore le mélange pendant 3 h, puis 2,97 g (2,69 ml ; 37,8 mmole) de chlorure d'acétyle dans 15 ml de toluène ont été ajoutés. Une dernière fraction constituée par 1,49 g (1,35 ml; 18,9 mmole) de chlorure d'acétyle dans 10 ml de toluène a enfin été additionnée pour compléter la réaction. Après 30 min, 200 ml d'eau ont été ajoutés à froid puis on a acidifié avec 100 ml d'une solution aqueuse d'$H_2SO_4$ à 10% et on a extrait à l'éther. Les phases organiques réunies ont été lavées avec de l'eau, une solution aqueuse à 5% de NaOH, une solution aqueuse saturée de NaCl puis séchées sur $Na_2SO_4$ et évaporées. On a ainsi obtenu après distillation (100-180°/66,5 Pa) 18,49 g d'un mélange de (3RS,4SR)-(E)-3-(1,3-butadiényl)-4-pentyl-2-oxétanone et (3RS,4RS)-(E)-3-(1,3-butadiényl)-4-pentyl-2-oxétanone (70:30 ; rend. 51%). Les deux constituants de ce mélange ont été séparés par chromatographie sur colonne remplie de $SiO_2$ (éluant : cyclohexane/acétate d'éthyle : 95/5).

Les caractères analytiques du (3RS,45R)-(E)-3-(1,3-butadiényl)-4-pentyl-2-oxétanone étaient les suivants :

| | |
|---|---|
| $^1$H-RMN(360MHz) : | 0,90(3H,s large) ; 1,20-1,80(8H,m); 4,38(1H,t,J=7); 4,60(1H,m); 5,19-(1H,d,J=10); 5,09(1H,d,J=15); 5,64(1H,dxd,J=14,5 et 7); 6,35(2H,m) δ ppm |
| $^{13}$C-RMN(360MHz) : | 13,9(q) ; 22,4(t) ; 24,8(t) ; 31,0(t) ; 31,4(t) ; 55,7(d) ; 76,2(d) ; 119,3(t) ; 120,6(d) ; 135,6(d) ; 137,3(d) ; 169,6(s) δ ppm |
| SM : | 150(18), 91(32), 79(100), 66(45), 41(28). |

Les caractères analytiques du (3RS,4RS)-(E)3-(1,3-butadiényl)-4-pentyl-2-oxétanone étaient les suivants :

$^{1}$H-RMN(360MHz) : 0,90(3H,t,J = env. 7,3) ; 1,23-1,97(8H,m) ; 3,88(1H,dxd, J = 8 et 4,5) ; 4,35-(1H,dxdxd,J = 7, 7 et 4,5) ; 5,18(1H,d,J = 10) ; 5,27(1H,d, J = 16) ; 5,73-(1H,dxd,J = 14,5 et 7) ; 6,30(2H,m) δ ppm

$^{13}$C-RMN(360MHz) : 13,9(q) ; 22,4(t) ; 24,6(t) ; 31,4(t) ; 34,2(t) ; 59,1(d) ; 78,3(d) ; 119,2(t) ; 123,4(d) ; 135,6(d) ; 168,9(s) δ ppm

SM : 150(16), 91(33), 79(100), 66(35), 41(22).

Le spectre IR du mélange des deux lactones était le suivant :

IR: 2925, 1810, 1680, 1600, 1460 $cm^{-1}$.

d. 18,49 g du mélange des lactones obtenues comme indiqué ci-dessus à la lettre c. dans 210 ml d'acétate d'éthyle ont été pyrolysés dans une colonne en quartz d'une longueur de 4 m sous une pression d'azote à 280° (débit : 1,3 ml/min). Le pyrolysat, récolté dans une trappe refroidie avec neige carbonique et acétone, a été évaporé et le résidu traité avec 100 mg de BHA puis distillé à 80-82°/5,32x$10^{2}$ Pa. On a obtenu ainsi 9,88 g (rend. : 69%) d'undéca-1,3,5-triène sous forme d'un mélange 70:30 d'undéca1,3E,5Z-triène et undéca-1,3E,5E-triène.

## Revendications

**1.** Procédé pour la préparation d'undéca-1,3,5-triène caractérisé en ce qu'on

a. additionne de l'hexanal au sel dilithié de l'acide sorbique de formule

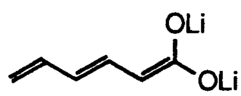

(I)

pour fournir un acide β-hydroxylique de formule

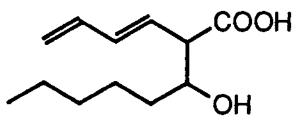

(II)

et

b. soumet ledit acide β-hydroxylique à une réaction d'élimination par traitement avec un réactif constitué par un N,N-diméthylformamidedialkylacétal ou par l'adduit constitué par la triphénylphosphine et l'ester diéthylique de l'acide azodicarboxylique,

ou

c. convertit ledit acide β-hydroxylique en la lactone de formule

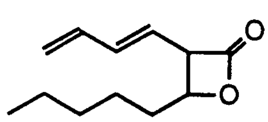

(III)

par traitement avec le chlorure d'acétyle, l'anhydride acétique, le chloroformiate de méthyle, le chlorure de méthylsulfonyle ou un chlorure d'arènesulfonyle en présence d'une base ;

et

d. soumet la lactone ainsi obtenue à un traitement thermique à une température comprise entre 250 et 300°C.

**2.** Procédé selon la revendication 1, caractérisé en ce que l'addition de l'hexanal au sel dilithié de l'acide sorbique de formule (I) s'effectue à une température inférieure à 10°C, de préférence à environ 0°C, pour fournir un acide β-hydroxylique dont le contenu en l'isomère *anti* est prépondérant.

**3.** Procédé selon la revendication 1, caractérisé en ce que l'addition de l'hexanal au sel dilithié de l'acide sorbique de formule (I) s'effectue à une température d'environ 50 ° C pour fournir un acide $\beta$-hydroxylique dont le contenu en l'isomère *syn* est prépondérant.

**4.** Procédé selon la revendication 1 caractérisé en ce qu'on utilise comme N,N-diméthylformamide-dialkylacétal le N,N-diméthylformamidediméthylacétal ou le N,N-diméthylformamide-dinéopentylacétal.

**5.** Procédé selon la revendication 1 caractérisé en ce qu'on utilise comme chlorure d'arènesulfonyle le chlorure de benzènesulfonyle.

**6.** Procédé selon la revendication 1 caractérisé en ce que la réaction d'élimination suivant l'étape b. s'effectue dans le diméthylformamide.

**7.** Procédé selon la revendication 1 caractérisé en ce que la conversion de l'acide $\beta$-hydroxylique en la lactone correspondante suivant l'étape c. s'effectue en présence d'une base organique constituée par une amine tertiaire.

**8.** Procédé selon la revendication 7 caractérisé en ce qu'on utilise comme amine tertiaire la triéthylamine.

**9.** Procédé selon la revendication 1 caractérisé en ce que le traitement thermique de la lactone suivant l'étape d. s'effectue à environ 280 ° C dans une atmosphère de gaz inerte.

**10.** Utilisation à titre d'ingrédient parfumant ou aromatisant d'undéca-1,3,5-triène obtenu par le procédé selon l'une des revendications 1 à 9.

**11.** L'acide (1RS,2SR)-2-(1-hydroxyhexyl)-3,5-hexadiénoïque ou l'acide (1RS,2RS)-2-(1-hydroxyhexyl)-3,5-hexadiénoïque.

**12.** La (3RS,4SR)-(E)-3-(1,3-butadiényl)-4-pentyl-2-oxétanone ou la (3RS,4RS)-(E)-3-(1,3-butadiényl)-4-pentyl-2-oxétanone.